# EUROPÄISCHE PATENTSCHRIFT

(11) **EP 1 282 400 B1**
(45) Veröffentlichungstag und Bekanntmachung des Hinweises auf die Patenterteilung: **14.12.2016**
(21) Anmeldenummer: 01940482.1
(22) Anmeldetag: 16.05.2001
(51) Int. Cl.: A61K 9/08, A61K 9/19, A61K 47/12, A61K 47/26, A61K 47/40

(54) **PHARMAZEUTISCHE DARREICHUNGSFORM FÜR PEPTIDE, VERFAHREN ZU DEREN HERSTELLUNG UND VERWENDUNG**
PHARMACEUTICAL FORM OF ADMINISTRATION FOR PEPTIDES, METHODS FOR ITS PRODUCTION AND USE
FORME D'ADMINISTRATION PHARMACEUTIQUE POUR DES PEPTIDES, SON PROCEDE DE PRODUCTION ET SON UTILISATION

(30) Priorität: 18.05.2000 DE 10024451
(43) Veröffentlichungstag der Anmeldung: 12.02.2003
(73) Patentinhaber: Æterna Zentaris GmbH, 60314 Frankfurt am Main (DE)
(72) Erfinder: BAUER, Horst, 91217 Hersbruck (DE); DAMM, Michael, 63322 Rödermark (DE); SARLIKIOTIS, Werner, GR-190 02 Peania (GR)
(86) Internationale Anmeldenummer: PCT/EP2001/005555
(87) Internationale Veröffentlichungsnummer: WO 2001/087265

(56) Entgegenhaltungen:
- EP-A- 0 175 506
- WO-A-96/07399
- WO-A-97/41836
- WO-A-98/25642
- WO-A-98/32423
- WO-A-98/42381
- S. ANNA SEILER: "ROTE LISTE 2000", 2000, ROTE LISTE SERVICE GMBH, FRANKFURT A M

## Beschreibung

Die Erfindung betrifft neue galenische Formen zur parenteralen Anwendung von zur Aggregation neigenden peptidischen LHRH-Analoga bzw. LHRH-Antagonisten sowie Verfahren zu deren Herstellung und Verwendung.

Es ist aus EP 0 299 402 bekannt, pharmazeutisch wirksame Decapeptide wie SB-030, SB-075(Cetrorelix) und SB-088 in Form ihrer pharmazeutisch akzeptablen, nicht toxischen Säureadditionssalze wie Hydrochloride, Hydrobromide, Sulfate, Phosphate, Fumarate, Gluconate, Tannate, Maleate, Acetate, Citrate, Benzoate, Succinate, Alginate, Pamoate, Ascorbate und Tartrate u.s.w. einzusetzen.
Aus JP 06321800-A ist ferner eine lyophilisierte Peptid- oder Proteinpräparation bekannt, die Gluconatsalze als Stabilisatoren enthält. In einem Beispiel enthält die Lösung 2,5% Magnesium Gluconat, wobei als Wirkstoff u.a. Vasopressin, LHRH und Insulin beschrieben sind.
Aus der Literatur ist unter anderem aus Powell, M.F., Pharmaceutical Research, 1258-1263(8) 1991; Dathe M., Int. J. Peptide Protein Res. 344-349(36) 1990, und Szejtli, J. Pharmaceutical Technology International 16-22, 1991, bekannt, dass Oligopeptide und zwar besonders solche mit endständiger Säureamidfunktion zur Gelbildung neigen.
Im EP 0 611 572 ist ein Herstellungsverfahren für ein Lyophilisat aus einem Peptid mit 3-15 Aminosäuren beschrieben, wonach 100-10 000 Gewichtsteile des Peptides in Essigsäure aufgelöst und mit Gerüststoffen wie Mannit versetzt sowie anschließend lyophilisiert werden, um ein sterilfiltriertes Lyophilisat des Peptides zu erhalten und Gelbildung zu vermeiden.
In der DE OS 195 42 873 sind kompliziert zusammengesetzte pharmazeutische Darreichungsformen in Form von Mikropartikeln beschrieben, wonach man ein ABA - Triblock - Copolymer verwendet, dessen A - Block ein Polymer aus Milch und Glykolsäure und dessen B Polymer eine Polyethylenglykolkette darstellt zusammen mit einem Zuschlagstoff aus der Gruppe der Serumproteine, Polyaminosäuren, Cyclodextrine, Cylodextrinderivate, Saccharide, Aminozucker, Aminosäuren, Detergentien oder Carbonsäuren sowie Gemischen dieser Stoffe. Die beschriebenen Mikropartikel sollen auch nach Einschluss geringer bzw. aggregationsempfindlicher Polypeptidmengen das Polypeptid über einen längeren Zeitraum kontinuierlich freisetzen.
In DD 141 996 wird die Herstellung von Arzneiformen des nativem LHRH beschrieben, die über einen längeren Zeitraum stabil sind und den Anforderungen an ein parenteral applizierbares Präparat entsprechen. Der Schwerpunkt ist hierbei die Verbesserung der Haltbarkeit dieser Präparationen (Seite 2, Zeilen 19-23). Über die Filtrierbarkeit der Lösungen wird keine Aussage gemacht. Darüber hinaus werden zur Verbesserung der Haltbarkeit auch Puffersubstanzen (auch Essigsäure) eingesetzt, um einen pH Bereich von pH 3,5 - 6,5 einzustellen. Das Problem, aus gelbildenen Peptidsalzen sterile Lyophilisate herzustellen, wird dort nicht gelöst.
In EP 0 175 506 wird eine wässrige Lösung des Peptides mit 1 N Essigsäure behandelt und danach lyophilisiert, um das Acetatsalz des Peptides zu erhalten. Somit ist der Gegenstand dieser Anmeldung die Synthese der Peptidsalze.
Es hat sich aber gezeigt, dass bei den bekannten Acetatsalzen der zur Aggregation neigenden Peptide, wie z.B. der LHRH-Antagonisten, die Herstellung steriler Lösungen zur parenteralen Anwendung mittels Filtration, speziell bei hohen Konzentrationen zwar möglich ist, sich jedoch Aggregate nach der Auflösung des Lyophilisates kurz vor der Injektion formen können. Die Aggregate führen nun zu einer konzentrationsabhängigen Erniedrigung der Bioverfügbarkeit ab einer Peptid-Konzentration von 0,5 mg/ml.
Das genannte Problem tritt nicht nur bei Injektionslösungen, die zwecks einer schnellen Wirkstofffreisetzung appliziert werden, auf, sondern wird auch bei Injektionspräparaten beobachtet, die eine verzögerte Freisetzung zeigen. So können Peptide, inkorporiert in Matrizes, welche die Wirkstofffreisetzung steuern sollen, aufgrund ihrer Neigung zur Aggregation eine unerwünscht niedrige Freisetzung aufweisen. So ist auch hier die Bioverfügbarkeit erniedrigt.

WO 98/32423 A1 beschreibt physiologisch aktive Peptide in Form von Mikropartikeln aus biologisch abbaubarem polymerem Material, das eine stetige und kontrollierte Freisetzung der aktiven Substanz gewährleistet sowie ein Verfahren zur Herstellung der verzögert freisetzenden Mikropartikel.
Als Peptide bzw. deren Salze werden LHRH-Agonisten oder LHRH-Antagonisten beschrieben.
Der Einsatz von Embonsäure oder Alkaliembonaten führt zu schwerlöslichen Embonatsalzen der Peptide.

Weiterhin lehrt WO 98/32423 A1 die Verwendung von α-Hydroxycarbonsäuren. Dieser Einsatz von α -Hvdroxycarbonsäuren in Form von Milchsäure/Glykolsäure-Polymeren bildet die Grundlage der biologisch abbaubaren Polymere.
Das Verfahren zur Herstellung läuft über ein Emulgieren mittels eines organischen Lösungsmittels und dessen anschließendem Entfernen.

Ausgehend von der Tatsache, dass die bevorzugte Applikation von pharmazeutisch wirksamen peptidischen LHRH-Antagonisten, beispielsweise Antarelix und Cetrorelix, die parenterale Arzneiform ist, bestand ein Bedarf an der Bereitstellung stabiler Injektionspräparate mit akzeptabler Bioverfügbarkeit, die sich günstig herstellen, sterilfiltrieren und konfektionieren lassen. Dies gilt insbesondere für Injektionspräparate in Form von rekonstituierten Lyophilisaten aus löslichen Peptidsalzen und für Mikropartikel, Mikrokapseln oder Implantate.
Dies ist umsomehr von Bedeutung in Anbetracht der immer mehr bekannt werdenden vielseitigen Anwendungsgebiete der LHRH-Antagonisten.
Eine breitere Auswahl parenteral, inbesondere subcutan injizierbarer, stabiler Peptidlösungen im Hinblick auf die schnell wachsenden Indikationsgebiete dieser Stoffklasse ist wünschenswert.

Es wurden nun pharmazeutische, zur parenteralen Anwendung geeignete Darreichungsform, die zur Aggregation neigende peptidische LHRH-Antagonisten in gelöster oder dispergierter Form enthalten, entwickelt, welche sich dadurch auszeichnen, dass die peptidische LHRH-Antagonisten in Form ihrer Acetat-, Gluconat-, Glucoronat,- Lactat-, Zitrat-, Ascorbat-, Benzoat- oder Phosphat-Salze vorliegen und dass diese Darreichungsform zusätzlich eine der Säuren, ausgewählt aus der Gruppe, bestehend aus Gluconsäure, Glucuronsäure, Galacturonsäure, Glucarsäure, Zitronensäure, Milchsäure, Ascorbinsäure und Aminosäuren als freie Säuren beinhalten können sowie ggf. weitere Zusatz- und Hilfsstoffe aus der Klasse der Säuren, oberflächenaktiven Substanzen, Polymere, Lipide oder Zucker.
Diese pharmazeutische Darreichungsformen können in Wasser oder in wäßrigen Lösungsmittelgemischen in gelöster oder dispergierter Form vorliegen.
Nach einer weiteren Ausführungsform der Erfindung können die pharmazeutischen Darreichungsformen auch in einem physiologisch verträglichen Öl, vorzugsweise mittelkettige Triglyceride (Neutralöle, Miglyol^{®}) bzw. Rizinusöl, Sesamöl, Baumwollsamenöl, Maisöl, Erdnussöl, Olivenöl, oder in Gemischen solcher Öle, in gelöster oder dispergierter Form vorliegen.
Bei den zum Einsatz gelangenden Peptiden handelt es sich um die LHRH-Antagonisten Antide, A-75998, Ganirelix und Nal-Glu-Antagonist, insbesondere jedoch um Cetrorelix-, und Antarelix.

Somit ist es möglich, die Aggregation des peptidische LHRH-Antagonisten zu unterdrücken und so die Anforderungen an ein Präparat mit guter Bioverfügbarkeit zu erfüllen, so den Arzneischatz zu bereichern und das bei einer effizienten galenischen Technologie.
Es wurde weiter überraschend gefunden, dass durch den Zusatz von Glucon-, Glucuron-, Zitronen-, Milch- bzw. Ascorbinsäure außerdem die Stabilität diverser Cetrorelix-Salze erheblich verbessert wird.
Erfindungsgemäß ist so die Herstellung und Konfektionierung sterilfiltrierter, stabiler Präparate problemlos möglich.
Vorteilhaft ist es, zusätzlich geeignete Hilfsstoffe zuzusetzen. Diese Hilfsstoffe können Säuren, oberflächenaktive Substanzen, Polymere, Lipide oder Zucker sein. Beispiele für Säuren sind Gluconsäure, Glucuronsäure, Galacturonsäure, Glucarsäure, Milch-, Zitronensäure, Ascorbinsäure und Aminosäuren. Als oberflächenaktive Substanzen können Polyäthylenglykol-12- (hydroxy)-stearat (Solutol^{®}), Polyoxyethylenrizinoleat (Cremophor^{®}), Polysorbate, Poloxamere, Phospholipide, Lecithine oder Benzalkoniumchlorid eingesetzt werden. Geeignete Polymere sind Albumine, Polyethylenglykole, Cellulosederivate, Stärkederivate oder Polyvinypyrrolidon. Beispiele für Zucker sind Cyclodextrine oder Cyclodextrinderivate. Auch sogenannte chaotrope Substanzen wie Harnstoff können als Zusatz- bzw. Hilfsstoffe dienen.

Das Einsatzgebiet der erfindungsgemäßen Zubereitungen liegt insbesondere in der Prävention und Therapie aller durch LHRH-Analoga, d. h. LHRH-Agonisten und LHRH-Antagonisten, beeinflußbaren Sexualhormon-abhängigen Zustände und Erkrankungen. Hierbei sind hervorzuheben:
Benigde Prostatahyperplasie, Prostatakarzinom, Pubertas Praecox, Hirsutismus, Endometriumhyperplasie sowie deren Begleiterscheinungen, Endometriumkarzinom, In vitro Fertilisation (IVF/COS/ART), Kontrazeption, Prämenstruelles Syndrom (PMS), Uterusmyomatose, Brustkrebs, Tubal Obstruktion (PTO), Ovarialkrebs, Uteruskarziom. Als LHRH-Antagonisten werden für die erfindungsgemäße Zusammensetzung folgende Substanzen beansprucht:
   Cetrorelix, Antarelix, Antide, A-75998, Ganirelix, der Nal-Glu Antagonist.

### Beispiel 1

Mittels Polarisations-Mikroskopie wurden Aggregationsuntersuchungen an Lösungen von verschiedenen Cetrorelix-Salzen ohne bzw. mit Zusatz von Hilfsstoffen durchgeführt.
Aggregierte Peptid-Lösungen zeigen im Polarisations-Lichtmikroskop mit gekreuzten Polarisatoren Bilder, die denen von flüssigkristallinen Strukturen sehr ähnlich sind. Im Gegensatz dazu ergeben aggregatfreie Peptid-Lösungen keine solchen Effekte.

**Tab. 1: Einfluss eines Gluconsäure-Zusatzes auf das Aggregationsverhalten von Cetrorelix-Acetat-Lösungen.**

| Konzentration Cetrorelix-acetat, mg/ml | Gluconsäure im Rekonstitutionsmedium, % | pH-Wert | Tage ohne Aggregation |
|---|---|---|---|
| | | | |
| 2,5 | 0 | 4,7 | 1 |
| 2,5 | 0,0071 | 4,5 | 2 |
| 2,5 | 0,071 | 3,7 | 2 |
| 2,5 | 0,71 | 3,1 | 12 |

Somit bewirkt der Zusatz von Gluconsäure eine Verbesserung der Stabilität von Cetrorelix-Acetat Lösungen, indem die Aggregation verzögert bzw. verhindert wird.

Weitere Versuche konzentrieren sich auf Cetrorelix-Gluconat ohne bzw. mit Zusatz von Gluconsäure. Die wichtigsten Ergebnisse sind in Tab. 2 zusammengefasst.

**Tab. 2: Aggregationsverhalten verschiedener Lösungen, die aus Bulkware von Cetrorelix-Gluconat hergestellt wurden.**

| Gluconsäure-Zusatz: | Ja | | Nein | |
|---|---|---|---|---|
| Konzentration Cetrorelix, mg/ml | PH | Tage ohne Aggregation | PH | Tage ohne Aggregation |
| | | | | |
| 2,5 | 3,0 | > 30 | | |
| | | | | |
| 5 | 3,6 | 4 | 4,8 | 1 |
| 5 | 3,8 | 4 | 4,7 | 1 |
| | | | | |
| 7,5 | 3,4 | 1 | 4,7 | 0 |
| 7,5 | 3,7 | 1 | 4,8 | 0 |

Cetrorelix-Gluconat bietet somit Vorteile im Vergleich zu dem Acetat-Salz. Der Zusatz von Gluconsäure erhöht die Haltbarkeit von Cetrorelix-Gluconat Lösungen.

Darüberhinaus wurde der stabilisierende Einfluss von Glucuronsäure auf Cetrorelix-Acetat-Lösungen und als weiteres Salz auch Cetrorelix-Glucuronat auf sein Aggregationsverhalten hin getestet. Die Ergebnisse sind in Tab. 3 zusammengefasst.

**Tab. 3: Aggregationsverhalten verschieden konzentrierter Lösungen von Cetrorelix-Acetat und Cetrorelix-Glucuronat ohne bzw. mit Zusatz von Glucuronsäure.**

| | Glucuronsäure-Zusatz: | Ja | | Nein | |
|---|---|---|---|---|---|
| Salzform | Konzentration Cetrorelix, mg/ml | pH | Tage ohne Aggregation | PH | Tage ohne Aggregation |
| | | | | | |
| Acetat | 2,5 | 3,0 | > 21 | 4,7 | 0 |
| | | | | | |
| Acetat | 5 | 3,0 | 0 | | |
| | | | | | |
| Glucuronat | 2,5 | 2,9 | > 30 | 4,5 | 3 |
| | | | | | |
| Glucuronat | 5 | 2,7 | > 30 | 4,6 | 0 |

Auch durch den Ersatz des Acetat-Salzes durch ein Glucuronat-Salz können signifikante Verbesserungen bezüglich der Aggregations-Stabilität von Cetrorelix-Lösungen ähnlich wie mit dem Gluconat-Salz erzielt werden. Durch den Zusatz von Glucuronsäure zu Cetrorelix-Glucuronat-Lösungen kann die Aggregationsstabilität dieser Lösungen noch weiter verbessert werden.

**Tab. 4: Aggregationsfreie Zeitdauer in Tagen von Cetrorelixacetat-Lösungen nach Zusatz von 10% a-Cyclodextrin, 20 % Hydroxypropyl-β-Cyclodextrin bzw. 20 % γ-Cyclodextrin.**

| Konzentration Cetrorelixacetat, mg/ml | α-Cyclodextrin | Hydroxypropyl-β-Cyclodextrin | γ-Cyclodextrin |
|---|---|---|---|
| 2,5 | 7 | 24 | 98+(168,182, 189) |
| 5 | 0 | 7 | 31 + (140, 147, 182) |
| 7,5 | 0 | 10 | 5 + (20, 20, 20) |
| 10 | 0 | 2 | 2+(4,4,4) |
| 15 | | 0 | 0 |

Durch den Zusatz von Hydroxypropyl-β-Cyclodextrin und besonders von γ-Cyclodextrin kann die Aggregationsstabilität von Cetrorelixacetat-Lösungen signifikant verbessert werden.

**Tab. 5: Aggregationsfreie Zeitdauer in Tagen von 2,5 mg/ml Cetrorelixgluconat-Lösungen nach Zusatz von α-Cyclodextrin, Hydroxypropyl-β-Cyclodextrin bzw. γ-Cyclodextrin.**

| Cyclodextrin-Typ | Konzentration Cyclodextrin, % | Tage ohne Aggregation |
|---|---|---|
| γ-Cyclodextrin | 20 | 182 |
| | 6,8 | 126 |
| Hydroxypropyl-β-Cyclodextrin | 20 | 189 |
| | 6,8 | 91 |
| α-Cyclodextrin | 10 | 140 |
| | 5 | 1 |

Durch den Zusatz von Hydroxypropyl-β-Cyclodextrin bzw. von γ-Cyclodextrin kann auch die Aggregationsstabilität von Cetrorelixgluconat-Lösungen signifikant verbessert werden.

**Tab.6: Aggregationsfreie Zeitdauer in Tagen von Cetrorelixacetat-Lösungen mit Zusatz von Polyvinylpyrrolidon (Kollidon^{®} 12 PF bzw. 17 PF).**

| Konzentration Cetrorelix, mg/ml | Konzentration an Kollidon^{®}, % | Tage ohne Aggregation mit Kollidon^{®} 12 PF | Tage ohne Aggregation mit Kollidon^{®} 17 PF |
|---|---|---|---|
| 2,5 | 0 | 0 | 0 |
| | 5 | 1 | 2 |
| | 10 | 1 | 2 |
| | 15 | 77 | 63 |
| | 20 | 84 | 98 |
| | | | |
| 5 | 15 | 0 | 1 |
| | 20 | 0 | 1 |

Auch duch den Zusatz verschiedener Polyvinylpyrrolidon-Typen kann die Aggregationsstabilität von Cetrorelixacetat-Lösungen signifikant verbessert werden.

**Tab. 7: Aggregationsverhalten von Cetrorelixacetat-Lösungen unter Zusatz verschiedener Hilfsstoffe beurteilt mittels Polarisations-Mikroskopie und nach dem optischen Erscheinungsbild (Aussehen).**

| Hilfsstoff | Konz. Hilfsstoff | Konz. Cetrorelix | Aggregation (Mikroskopie) | Aussehen |
|---|---|---|---|---|
| Solutol^{®} HS 15 | 5,00 % | 2,5 mg/ml | ja, nach 14 Tagen | klare Lösung |
| | 10,00% | 2,5 mg/ml | ≥112 Tage ohne Aggregation | klare Lösung |
| | 20,00% | 2,5 mg/ml | ≥ 112 Tage ohne Aggregation | klare Lösung |
| Cremophor^{®} EL | 5,00 % | 2,5 mg/ml | ja, nach 10 Tagen | klare Lösung |
| | 10,00 % | 2,5 mg/ml | ≥ 112 Tage ohne Aggregation | klare Lösung |
| | 20,00% | 5 mg/ml | ja, nach 1 Tag | klar, viskos |
| L-Glutaminsäure | 0,80 % | 2,5 mg/ml | ja, nach 2 Tagen | klare Lösung, pH 3,8 |
| Glucarsäure | 2,50% | 2,5 mg/ml | ≥ 12 Tage ohne Aggregation | klare Lösung, pH 2,5 |
| Galacturonsäure | 2,50% | 2,5 mg/ml | ≥ 12 Tage ohne Aggregation | klare Lösung, pH 2,6 |

### Beispiel 2

Cetrorelix-Bulkware wird in 30 %iger Essigsäure in einer Konzentration von 10 mg/ml gelöst und mit einer wässrigen Lösung der Zusatzstoffe auf eine Endkonzentration von 1 mg/ml Peptid in 3 % Essigsäure verdünnt. Diese Lösung wird dann sterilisiert und lyophilisiert (5 mg pro Vial).

Nach Rekonstitution dieser Lyophilisate werden die Lösungen (2,5 mg/ml Cetrorelix) in folgenden Tests auf Aggregatbildung und Freisetzungsverhalten hin untersucht:
- Polarisationsmikroskopie (Pol. Mik.): Tage ohne Aggregation.
- Filtrierbarkeit in %:
   Cetrorelix-Lösungen werden nach einem standarisierten Verfahren hergestellt und durch 0,22 µm bzw 0,45 µm Filter mittels Zentrifugation filtriert. Die Konzentration an Cetrorelix im Filtrat wird per HPLC bestimmt und als %-Wert, bezogen auf die Ausgangskonzentration vor Filtration, angegeben (Filtrierbarkeit in %).
- In-vitro-Freisetzung Kurzform (RRS, Release in Ringer Solution):
   % freigesetzt nach 1 h und nach 6 h.

   Das in-vitro Freisetzungsverhalten wird in einem Durchflußverfahren mit Ringer-Lösung als Medium bei 37°C bestimmt. Die Konzentrationsmessung erfolgt per HPLC. Cetrorelix-Proben, entsprechend 10 mg Cetrorelixbase, werden in die Durchflußzelle eingewogen, mit 4 ml Wasser versetzt und 10 min gerührt. Nach Zugabe von 6 ml Ringer-Lösung zur Probe wird unter Rühren gleichmäßig mit einem Fluß von 0,5 ml/min Ringer-Lösung durch die Durchflußzelle gepumpt.
- Ratten-Tierversuch: Cetrorelix-Restanteil im Muskel in % der applizierten Dosis 168 h nach Injektion.

In der Tabelle 8a sind einige hergestellte Cetrorelixacetat-Lyophilisatchargen und die entsprechenden Testergebnisse von daraus hergestellten 2,5 mg/ml Cetrorelixacetat-Lösungen aufgeführt.

**Tab. 8a**

| Cetrorelixacetat-Lyophilisatchargen (5mg)... | Pol. Mik., Tage o. Aggr. | 0,22 µm filtrierbar | RRS , [%] nach | | Ratte % i.m. nach |
|---|---|---|---|---|---|
| Hilfsstoffe | | [%] | 1h | 6h | 168 h |
| Nur Mannit (= Kontrolle) | 0 | | | | ca. 55 |
| Solutol ^{®} / Mannit | 48 | 100 | | | |
| Cremophor ^{®} / Mannit | 46 | 101 | | | |
| Solutol ^{®} / Alanin | 16 | 98 | 17 | 24 | |
| Solutol ^{®} / Alanin / Gluconsäure | 19 | 101 | 57 | 68 | 5,7 |
| Solution/ Mannit / Gluconsäure | >45 | 100 | 84 | 88 | 3,8 |
| Cremophor ^{®} / Mannit / Gluconsäure | >45 | 101 | | | |
| Solutol ^{®} / Tryptophan / Mannit | Unmögl. | | | | |
| Solutol ⁹ / Tryptophan / Gluconsäure | 6 | | | | 9,6 |
| Cyclodextrin Molverh. 1:10/ Mannit | 2 | 101 | 16 | 27 | 10 |
| Cyclodextrin Molverh. 1:10 / Mannit / Gluconsäure | >45 | 102 | 68 | 74 | |
| Cyclodextrin Molverh. 1:30 / Mannit | 17 | 100 | 68 | 76 | |
| Cyclodextrin Molverh. 1:10 / Alanin / Gluconsäure | 5 | 101 | 39 | 52 | 6,3 |
| Mannit / Citronensäure | 1 | 102 | 45 | 53 | |
| Solutol ^{®} / Mannit / Citronensäure | >36 | 100 | 84 | 91 | 7,4 |
| Solutol ^{®} / Alanin / Citronensäure | 1 | 99 | 47 | 54 | |
| Solutol ^{®} / Glycin | >36 | 97 | 24 | 31 | |
| Solutol ^{®} / Harnstoff | 21 | 100 | 32 | 40 | |
| Solutol ^{®} / Glycin / Gluconsäure | >36 | 99 | 82 | 89 | |
| Solutol ^{®} / Harnstoff / Gluconsäure | >36 | 100 | | | |
| Cremophor ^{®}/ Alanin / Gluconsäure | (36) | | | | |
| Cremophor ^{®} / Harnstoff / Gluconsäure | (36) | | | | |
| Pluronic ^{®} F127 / Mannit | 1 | | | | |
| 5% Tween ^{®} 80 / Mannit | >16 | | | | |
| Polyethylenglykol 4000 / Mannit | 1 | | | | |
| Dextran / Mannit | 1 | | | | |
| Phenylquecksilberacetat / Mannit | 2 | | | | |

An den aufgeführten Beispielen ist ersichtlich, dass mit einer Vielzahl der getesteten Hilfsstoffe aus verschiedenen Substanzgruppen (oberflächenaktive Substanzen, Säuren, Aminosäuren, Polymere, Zucker, Zuckeralkohole, Cyclodextrine, Konservierungsmittel) einzeln oder mit Gemischen dieser Hilfsstoffe stabilisierende Effekte in vitro (Polarisationsmikroskopie, Filtrierbarkeit, in vitro-Freisetzung) und in vivo erzielt werden können. Diese verringerte Aggregationstendenz und somit verbesserte in vitro Wirkstofffreisetzung führt auch im Ratten-Experiment zu verbesserten Bioverfügbarkeit des Peptidwirkstoffes und damit zu reduzierten Restgehalten im Rattenmuskel.

Weitere in vitro- und in vivo-Daten von Chargen mir verschiedenen Cetrorelix-Salzen ohne bzw. mit Zusatz von stabilisierenden Hilfsstoffen sind in folgender Tab. 8b aufgelistet.

**Tab. 8b:**

| Cetrorelix-Salze (mit Wasser rekonstituiert) | Konz. Cetroreli x aus Lyo | Pol.Mik Tage o. Aggr. | RRS, [%] nach | | Ratte % i.m. nach |
|---|---|---|---|---|---|
| Hilfsstoffe | mg/ml | | 1h | 6h | 168 h |
| - Acetat | 2,5 | 0 | 12 | 24,5 | ca. 55 |
| - Acetat | 2,5 | 0 | 13 | 35,9 | ca. 55 |
| - Acetat | 5 | 0 | 10 | 35 | |
| - Acetat mit Gluconsäure rekonstituiert | 2,5 | 18 | 50 | 63,2 | 15,2 |
| - Acetat + Kollidon® 12 PF | 2,5 | 84 | 15 | 43,4 | 20,2 |
| - Acetat + Kollidon ® 17 PF | 2,5 | 98 | 22 | 50,6 | |
| - Acetat + Benzalkoniumchlorid | 2,5 | | 6,3 | 30,3 | |
| - Acetat + Phospholipide | 2,5 | | 7,3 | 23,3 | |
| - Acetat + γ-Cyclodextrin (1:10) | 2,5 | | 22,6 | 44,5 | 10 |
| - Acetat + γ-Cyclodextrin (1:30) | 2,5 | | 28 | 56,7 | |
| - Acetat + γ-Cyclodextrin (1:50) | 2,5 | | 35,1 | 56,6 | |
| - Acetat + γ-Cyclodextrin (1:90) | 2,5 | > 168 | 34,5 | 60,2 | 3,6 |
| - Acetat + γ-Cyclodextrin (1:90) | 5 | 140 | 19 | 47,8 | |
| - Acetat + γ-Cyclodextrin (1:90) | 7,5 | 20 | | | |
| - Acetat + γ-Cyclodextrin (1:90) | 10 | 4 | | | 45,2 |
| - Acetat mit Gluconsäure rekonstituiert | 15 | | | | 49,1 |
| - Gluconat | 2,5 | | 18 | 45,3 | |
| - Gluconat | 2,5 | | 11,3 | 46 | |
| - Gluconat mit Gluconsäure rekonstituiert | 2,5 | | 77,5 | 83,6 | |
| - Citrat | 15 | - | 9 | 20,3 | |
| - Lactat | Bulk | | 20 | 55,2 | |
| - Embonat | 15 | - | 13 | 43 | |

### Beispiel 3

Cetrorelix-Formulierungen, die weniger / langsamer zur Aggregation neigen (bessere Filtrierbarkeit/Polarisationsmikroskopie) und schnellere in-vitro Freisetzung in Ringer-Lösung zeigen, fallen durch ihren geringeren Cetrorelix-Restgehalt nach 168 h im Rattenmuskel-Experiment auf. Von solchen Formulierungen erwartet man eine höhere Bioverfügbarkeit.

Einige Ergebnisse von Rattenmuskel-Experimenten sind bereits in den Tabellen 8a und 8b aufgelistet.

Bei den in Tabelle 9 gezeigten weiteren Rattenmuskel-Experimenten wurde neben dem Restgehalt im Muskel noch der Cetrorelix-Anteil im Plasma bestimmt. Auch anhand dieser Daten wird der stabilisierende Einfluß der getesteten Hilfsstoffe deutlich. Außerdem konnte durch den Ersatz des Acetat-Salzes durch andere Salzformen des Cetrorelix eine verbesserte Bioverfügbarkeit und einhergehend damit eine reduzierte Restmenge im Rattenmuskelexperiment erreicht werden.

**Tab. 9.**

| Substanz | Dosis | Cetrorelix-Konzentration der Inj. Lsg. | Cetrorelixanteil im Muskel (168 h) | Cetrorelixanteil im Plasma |
|---|---|---|---|---|
| (Cetrorelix-) | (mg/kg) | (mg/ml) | % der Dosis | % der Dosis |
| Acetat+Solutol®+Alanin+ Gluconsäure | 1,5 | 2,5 | 5,7 | |
| Acetat +Solutol®+ Tryptophan+Gluconsäure | 1,5 | 2,5 | 9,6 | |
| Acetat + Cyclodextrin 1:10 | 1,5 | 2,5 | 10,0 | 83,4 |
| Acetat + Cyclodextrin 1:10, Alanin, Gluconsäure | 1,5 | 2,5 | 6,3 | 81,8 |
| Acetat + Solutol ® + Gluconsäure | 1,5 | 2,5 | 3,8 | |
| Acetat + Solutol ® +Citronsäure | 1,5 | 2,5 | 7,4 | |
| | | | | |
| Acetat | 1,5 | 3 | 55,1 | 92,2 |
| Acetat in Miglyol ® | 1,5 | 3 | 22,3 | 74,2 |
| Acetat+Benzaloniumchlor id | 1,5 | 3 | 76,9 | 39,8 |
| Acetat +20% Cyclodextrin | 1,5 | 3 | 3,6 | 106,2 |
| Acetat+20% Kollidon ® | 1,5 | 3 | 20,2 | 88,4 |
| Acetat+Glucuronsäure | 1,5 | 3 | 23,6 | 106,1 |
| Acetat+Gluconsäure | 1,5 | 3 | 15,2 | 95,5 |
| | | | | |
| Acetat+20% Cyclodextrin | 3,0 | 10 | 45,2 | 60,9 |
| | | | | |
| Acetat | 3,0 | 15 | 56,5 | 28,7 |
| Acetat in Miglyol® | 3,0 | 15 | 24,2 | 57,2 |
| Acetat+0,025% Benzalkon. | 3,0 | 15 | 10,5 | 21,4 |
| Acetat + Glucuronsäure | 3,0 | 15 | 78,1 | 43,8 |
| Acetat + Gluconsäure | 3,0 | 15 | 49,1 | 45,5 |
| | | | | |
| Gluconat | 1,5 | 15 | 37,9 | 46,9 |
| Gluconat in Mannit | 1,5 | 3 | 24,6 | 58,0 |
| Gluconat in Mannit | 1,5 | 3 | 25,4 | 75,2 |
| Gluconat in Miglyol® | 1,5 | 3 | 28,8 | 46,3 |
| Gluconat in Gluconsäure | 1,5 | 3 | 13,2 | 120,0 |
| Gluconat in Gluconsäure | 3,0 | 3 | 29,2 | |
| Gluconat in Gluconsäure | 3,0 | 15 | 43,5 | 74,2 |
| | | | | |
| Glucuronat | 1,5 | 3 | 16,5 | 78,6 |
| Glucuronat | 3,0 | 15 | 18,8 | |
| Lactat | 3,0 | 15 | 33,2 | 72,1 |
| Lactat | 1,5 | 3 | 30,7 | 67,1 |
| Citrat-Lyo/a | 1,5 | 3 | 22,8 | 36,6 |
| Citrat in Miglyol® | 1,5 | 3 | 14,8 | 53,1 |
| Base | 1,5 | 3 | 27,2 | 122,2 |
| Base in Miglyol® | 1,5 | 3 | 38,9 | 55,9 |
| Benzoat in Mannit | 1,5 | 3 | 34,2 | 32,7 |
| Benzoat in Miglyol® | 1,5 | 3 | 33,1 | 21,1 |
| Phosphat | 1,5 | 3 | 32,9 | 22,6 |

## Patentansprüche

1. Pharmazeutische, zur parenteralen Anwendung geeignete Darreichungsform, die zur Aggregation neigende Peptide in gelöster oder dispergierter Form enthält, wobei es sich bei den Peptiden um peptidische LHRH-Antagonisten ausgewählt aus der Gruppe bestehend aus Cetrorelix, Antarelix, Antide, A-75998, Ganirelix und Nal-Glu Antagonist handelt, **dadurch gekennzeichnet, dass** die Peptide in Form ihrer Acetat-, Gluconat-, Glucuronat-, Lactat-, Zitrat-, Ascorbat-, Benzoat- oder Phosphat-Salze vorliegen, und dass diese Darreichungsform zusätzlich eine der Säuren ausgewählt aus der Gruppe bestehend aus Gluconsäure, Glucuronsäure, Zitronensäure, Milchsäure, und Ascorbinsäure, als freie Säuren, sowie ggf. weitere Zusatz- und Hilfsstoffe aus der Klasse der Säuren, oberflächenaktiven Substanzen, Polymere, Lipide oder Zucker beinhaltet.

2. Pharmazeutische Darreichungsform zur parenteralen Anwendung nach Anspruch 1, wobei die Darreichungsform in Wasser oder in wässrigern Lösungsmittelgemischen in gelöster oder dispergierter Form vorliegt.

3. Pharmazeutische Darreichungsform zur patrenteralen Anwendung nach Anspruch 1, wobei die Darreichungsform in einem physiologisch verträglichen Öl, vorzugsweise mittelkettige Triglyceride, Rizinusöl, Sesamöl, Baumwollsamenöl, Maisöl, Erdnussöl, Olivenöl oder in Gemischen solcher Öle, in gelöster oder dispergierter Form vorliegt.

4. Pharmazeutische Darreichungsform nach einem der Ansprüche 1 bis 3 **dadurch gekennzeichnet, dass** als Säuren in der Hilfsstoffunktion Gluconsäure, Glucuronsäure, Galacturonsäure, Glucarsäure, Milchsäure, Zitronensäure, Ascorbinsäure und Aminosäuren eingesetzt werden.

5. Pharmazeutische Darreichungsform nach einem der Ansprüche 1 bis 3 **dadurch gekennzeichnet, dass** als oberflächenaktive Substanzen Polyäthylenglykol-12-(hydroxy)-stearat, Polyoxyethylenrizinoleat, Polysorbate, Poloxamere, Phospholipide, Lecitine oder oberflächenaktive Substanzen in Form von Konservierungsmitteln, vorzugsweise Benzalkoniumchlorid oder Phenylquecksilberacetat, eingesetzt werden.

6. Pharmazeutische Darreichungsform nach einem der Ansprüche 1 bis 3, **dadurch gekennzeichnet, dass** als Polymere Albumine, Polyethylenglykole, Cellulosederivate, Stärkederivate oder Polyvinylpyrroliden eingesetzt werden.

7. Pharmazeutische Darreichungsform nach einem der Ansprüche 1 bis 3, **dadurch gekennzeichnet, dass** als Zucker Cyclodextrine oder dessen Derivate sowie Zuckeralkohole eingesetzt werden.

8. Pharmazeutische Darreichungsform nach Anspruch 1 bis 3 **dadurch gekennzeichnet, daß** als Hilfsstoff Harnstoff oder andere chaotrope Substanzen eingesetzt werden.

9. Pharmazeutische Darreichungsform zur parenteralen Anwendung nach einem der Ansprüche 1, 2, **dadurch gekennzeichnet, dass** die Peptidsalze der Essigsäure, Gluconsäure, Glucuronsäure, Milchsäure, Zitronensäure bzw. der Ascorbinsäure in Lösungen in einer Konzentration von höher als 0,5 mg/ml vorliegen.

10. Pharmazeutische Darreichungsform zur parenteralen Anwendung nach einem der Ansprüche 1, 2 und 9, **dadurch gekennzeichnet, dass** als Peptide Cetrorelix oder Antarelix in Lösungen in einer Konzentration von höher als 0,5 mg/ml eingesetzt werden.

11. Pharmazeutische Darreichungsform nach einem der Ansprüche 1 bis 10 **dadurch gekennzeichnet, dass** die Wirkstofffreisetzung durch den Einsatz von Polymeren, vorzugsweise Homo- oder Copolymeren der Milch- und Glykolsäure, retardiert wird.

12. Pharmazeutische Darreichungsform nach einem der Ansprüche 1 bis 11, **dadurch gekennzeichnet, daß** es sich bei den zur parenteralen Anwendung geeigneten Darreichungsformen um Injektionspräparate handelt, welche insbesondere zur subcutanen Injektion geeignet sind.

13. Pharmazeutische Darreichungsform zur parenteralen Anwendung nach einem der Ansprüche 1, 2, und 9, **dadurch gekennzeichnet, daß** der LHRH Antagonist Cetrorelix ist, daß Cetrorelix in Form seines Acetat-, Gluconat-, oder Glucuronat-Salzes vorliegt, daß diese Darreichungsform zusätzlich Gluconsäure oder Glucuronsäure als freie Säure sowie ggf. weitere Zusatz- oder Hilfsstoffe enthält, und in Wasser oder in wässriger Lösungsmittelgemischen in gelöster oder dispergierter Form vorliegt.

14. Pharmazeutische Darreichungsform zur parenteralen Anwendung nach Anspruch 13, **dadurch gekennzeichnet, daß** die weiteren Zusatz- oder Hilfsstoffe ausgewählt sind aus der Gruppe umfassend Mannit, Polyäthylenglykol-12-(hydroxy)-stearat, Polyoxyethylenrizinoleat, Glycin, Citronensäure, und Cyclodextrin, wobei Mannit bevorzugt ist.

15. Pharmazeutische Darreichungsform zur parenteralen Anwendung nach Anspruch 13 oder 14, **dadurch gekennzeichnet, daß** zur Herstellung der Darreichungsform ein Lyophilisat, enthaltend das Cetrorelix-Salz sowie ggf. weitere Zusatz- oder Hilfsstoffe, mit einem wäßrigen, Gluconsäure oder Glucuronsäure als freie Säure enthaltenden Medium rekonstituiert wird.

16. Verfahren zur Herstellung einer pharmazeutischen Darreichungsform nach Anspruch 1, **dadurch gekennzeichnet, dass** man
(a) durch Umsalzen von Peptidsalzen mit Essigsäure, Glucuronsäure, Gluconsäure, Milchsäure, Zitronensäure oder Ascorbinsäure die entsprechenden Salze im stöchiometrischen Verhältnis herstellt,
(b) in Wasser für Injektionszwecke löst,
(c) ggf. mit Hilfsstoffen gemäss den Ansprüchen 4 bis 8 versetzt,
(d) anschliessend sterilfiltriert, in Injektionsflaschen abfüllt und lyophilisiert, und
(e) das Lyophilisat vor der parenteralen Applikation rekonstituiert.

17. Verfahren zur Herstellung einer pharmazeutischen Darreichungsform nach Anspruch 11, **dadurch gekennzeichnet, dass** man
(a) durch Umsalzen von Peptidsalzen mit Essigsäure, Glucuronsäure, Gluconsäure, Milchsäure, Zitronensäure oder Ascorbinsäure die entsprechenden Salze im stöchiometrischen Verhältnis herstellt,
(b) diese Salze in an sich bekannter Weise in verzögert freisetzende Mikropartikel aus Homo- oder Copolymeren aus Milch- und Glykolsäure einarbeitet, und
(c) diese Mikropartikel in einem physiologisch verträglichen Medium für Injektionszwecke suspendiert.

18. Verwendung eines peptidischen LHRH Antagonisten zur Herstellung einer, zur parenteralen Anwendung geeigneten, pharmazeutischen Darreichungsform nach einem der Ansprüche 1 bis 9 sowie 11 und 12 zur Prävention und Therapie von Sexualhormon-abhängigen, gutartigen und bösartigen Erkrankungen.

19. Verwendung nach Anspruch 18, **dadurch gekennzeichnet, dass** der LHRH Antagonist ausgewählt ist aus der Gruppe bestehend aus Cetrorelix, Antarelix, Antide, A-75998, Ganirelix und Nal-Glu Antagonist.

20. Verwendung von Cetrorelix zur Herstellung einer, zur parenteralen Anwendung geeigneten, pharmazeutischen Darreichungsform nach einem der Ansprüche 13 bis 15 zur Prävention und Therapie von Sexualhormon-abhängigen, gutartigen und bösartigen Erkrankungen.

21. Verwendung nach einem der Ansprüche 18 bis 20, **dadurch gekennzeichnet, daß** die Sexualhormon-abhängigen, gutartigen und bösartigen Erkrankungen ausgewählt sind aus der Gruppe bestehend aus beninger Prostatahyperplasie, Prostatakarzinom, Pubertas Praecox, Hirsutismus, Endometriumhyperplasie sowie deren Begleiterscheinungen, Endometriumkarzinom, In-vitro-Fertilation (IVF/COS/ART). Kontrazeption, Prämenstruelles Syndrom (PMS), Uterusmyomatose, Brustkrebs, Tubal Obstruction (PTO), Ovarialkrebs und Uteruskarzinom.

## Claims

1. Pharmaceutical administration form suitable for parenteral administration, which contains peptides prone to aggregation in dissolved or dispersed form, wherein the peptides are peptidic LHRH antagonists selected from the group consisting of cetrorelix, antarelix, antide, A-75998, ganirelix and Nal-Glu antagonist, **characterized in that** the peptides are present in the form of their acetate, gluconate, glucuronate, lactate, citrate, ascorbate, benzoate or phosphate salts, and that this administration form additionally contains one of the acids selected from the group consisting of gluconic acid, glucuronic acid, citric acid, lactic acid and ascorbic acid as free acids and, if appropriate, further additives and excipients from the class consisting of the acids, surface-active substances, polymers, lipids or sugars.

2. Pharmaceutical administration form for parenteral administration according to Claim 1, the administration form being present in dissolved or dispersed form in water or in aqueous solvent mixtures.

3. Pharmaceutical administration form for parenteral administration according to Claim 1, the administration form being present in dissolved or dispersed form in a physiologically tolerable oil, preferably medium-chain triglycerides, castor oil, sesame oil, cottonseed oil, maize oil, peanut oil, olive oil or in mixtures of such oils.

4. Pharmaceutical administration form according to one of Claims 1 to 3, **characterized in that** the acids employed in the excipient function are gluconic acid, glucuronic acid, galacturonic acid, glucaric acid, lactic acid, citric acid, ascorbic acid and amino acids.

5. Pharmaceutical administration form according to one of Claims 1 to 3, **characterized in that** the surface-active substances employed are polyethylene glycol 12-(hydroxy)stearate, polyoxyethylene ricinoleate, polysorbates, poloxamers, phospholipids, lecithins or surface-active substances in the form of preservatives, preferably benzalkonium chloride or phenylmercury acetate.

6. Pharmaceutical administration form according to one of Claims 1 to 3, **characterized in that** the polymers employed are albumins, polyethylene glycols, cellulose derivatives, starch derivatives or polyvinylpyrrolides.

7. Pharmaceutical administration form according to one of Claims 1 to 3, **characterized in that** the sugars employed are cyclodextrins or derivatives thereof, and sugar alcohols.

8. Pharmaceutical administration form according to Claims 1 to 3, **characterized in that** urea or other chaotropic substances are employed as excipient.

9. Pharmaceutical administration form for parenteral administration according to either of Claims 1 and 2, **characterized in that** the peptide salts of acetic acid, gluconic acid, glucuronic acid, lactic acid, citric acid or ascorbic acid are present in solutions in a concentration of higher than 0.5 mg/ml.

10. Pharmaceutical administration form for parenteral administration according to one of Claims 1, 2 and 9, **characterized in that** the peptides employed in solutions in a concentration of higher than 0.5 mg/ml are cetrorelix or antarelix.

11. Pharmaceutical administration form according to one of Claims 1 to 10, **characterized in that** the release of active compound is delayed by the use of polymers, preferably homo- or copolymers of lactic and glycolic acid.

12. Pharmaceutical administration form according to one of Claims 1 to 11, **characterized in that** the administration forms suitable for parenteral administration are injection preparations which are suitable in particular for subcutaneous injection.

13. Pharmaceutical administration form for parenteral administration according to one of Claims 1, 2 and 9, **characterized in that** the LHRH antagonist is cetrorelix, **in that** cetrorelix is present in the form of its acetate, gluconate or glucuronate salt, **in that** this administration form additionally comprises gluconic acid or glucuronic acid as free acid and also, optionally, further additives or excipients, and is present in water or in aqueous solvent mixtures in dissolved or dispersed form.

14. Pharmaceutical administration form for parenteral administration according to Claim 13, **characterized in that** the further additives or excipients are selected from the group encompassing mannitol, polyethylene glycol 12-(hydroxy)stearate, polyoxyethylene ricinoleate, glycine, citric acid and cyclodextrin, preferably mannitol.

15. Pharmaceutical administration form for parenteral administration according to Claim 13 or 14, **characterized in that** for the production of the administration form, a lyophilizate comprising the cetrorelix salt and also, optionally, further additives or excipients is reconstituted with an aqueous medium comprising gluconic acid or glucuronic acid as free acid.

16. Process for the production of a pharmaceutical administration form according to Claim 1, **characterized in that**,
(a) by double decomposition of peptide salts with acetic acid, glucuronic acid, gluconic acid, lactic acid, citric acid or ascorbic acid, the corresponding salts are prepared in a stoichiometric ratio,
(b) dissolved in water for injection,
(c) if appropriate mixed with excipients according to Claims 4 to 8,
(d) then sterile-filtered, dispensed into injection vials and lyophilized, and
(e) the lyophilizate is reconstituted prior to the parenteral administration.

17. Process for the production of a pharmaceutical administration form according to Claim 11, **characterized in that**,
(a) by double decomposition of peptide salts with acetic acid, glucuronic acid, gluconic acid, lactic acid, citric acid or ascorbic acid, the corresponding salts are prepared in a stoichiometric ratio,
(b) these salts are incorporated in a manner known per se into delayed-release microparticles of homo- or copolymers of lactic and glycolic acid, and
(c) these microparticles are suspended in a physiologically tolerable medium for injection.

18. Use of a peptidic LHRH antagonist for producing a pharmaceutical administration form suitable for parenteral administration, according to one of Claims 1 to 9 and also 11 and 12, for the prevention and therapy of sex hormone-dependent, benign and malignant diseases.

19. Use according to Claim 18, **characterized in that** the LHRH antagonist is selected from the group consisting of cetrorelix, antarelix, antide, A-75998, ganirelix and Nal-Glu antagonist.

20. Use of cetrorelix for the production of a pharmaceutical administration form suitable for parenteral administration according to one of Claims 13 to 15 for the prevention and therapy of sex hormone-dependent, benign and malignant diseases.

21. Use according to one of Claims 18 to 20, **characterized in that** the sex hormone-dependent, benign and malignant diseases are selected from the group consisting of benign prostate hyperplasia, carcinoma of the prostate, precocious puberty, hirsutism, endometrial hyperplasia and its accompanying symptoms, endometrial carcinoma, in-vitro fertilization (IVF/COS/ART), contraception, premenstrual syndrome (PMS), uterine myomatosis, breast cancer, tubal obstruction (PTO), ovarian cancer and carcinoma of the uterus.

## Revendications

1. Forme galénique pharmaceutique, appropriée pour l'utilisation parentérale, qui contient des peptides, présentant une tendance à l'agrégation, sous forme dissoute ou dispersée, les peptides étant des antagonistes peptidiques de la LH-RH, choisis dans le groupe constitué par le cétrorélix, l'antarélix, l'antide, l'A-75998, le ganirélix et l'antagoniste Nal-Glu, **caractérisée en ce que** les peptides se trouvent sous forme de leurs sels d'acétate, de gluconate, de glucuronate, de lactate, de citrate, d'ascorbate, de benzoate ou de phosphate et **en ce que** cette forme galénique contient en outre un des acides choisis dans le groupe constitué par l'acide gluconique, l'acide glucuronique, l'acide citrique, l'acide lactique et l'acide ascorbique, sous forme d'acides libres, ainsi que le cas échéant d'autres additifs et adjuvants de la classe des acides, des substances tensioactives, des polymères, des lipides ou des sucres.

2. Forme galénique pharmaceutique pour l'utilisation parentérale selon la revendication 1, la forme galénique se trouvant sous forme dissoute ou dispersée dans de l'eau ou dans des mélanges aqueux de solvants.

3. Forme galénique pharmaceutique pour l'utilisation parentérale selon la revendication 1, la forme galénique se trouvant sous forme dissoute ou dispersée dans une huile physiologiquement acceptable, de préférence des triglycérides à chaîne moyenne, l'huile de ricin, l'huile de sésame, l'huile de graines de coton, l'huile de maïs, l'huile d'arachide, l'huile d'olive, ou dans des mélanges de ces huiles.

4. Forme galénique pharmaceutique selon l'une quelconque des revendications 1 à 3, **caractérisée en ce qu'**on utilise, comme acides dans la fonction adjuvante, l'acide gluconique, l'acide glucuronique, l'acide galacturonique, l'acide glucarique, l'acide lactique, l'acide citrique, l'acide ascorbique et des aminoacides.

5. Forme galénique pharmaceutique selon l'une quelconque des revendications 1 à 3, **caractérisée en ce qu'**on utilise, comme substances tensioactives, le 12-(hydroxy)-stéarate de polyéthylèneglycol, le ricinoléate de polyoxyéthylène, des polysorbates, des poloxamères, des phospholipides, des lécithines ou des substances tensioactives sous forme d'agents de conservation, de préférence le chlorure de benzalconium ou l'acétate de phénylmercure.

6. Formule galénique pharmaceutique selon l'une quelconque des revendications 1 à 3, **caractérisée en ce qu'**on utilise, comme polymères, l'albumine, des polyéthylèneglycols, des dérivés de cellulose, des dérivés d'amidon ou la polyvinylpyrrolidone.

7. Formule galénique pharmaceutique selon l'une quelconque des revendications 1 à 3, **caractérisée en ce qu'**on utilise, comme sucres, des cyclodextrines ou leurs dérivés ainsi que des alcools de sucre.

8. Forme galénique pharmaceutique selon la revendication 1 à 3, **caractérisée en ce qu'**on utilise, comme adjuvants, de l'urée ou d'autres substances chaotropiques.

9. Forme galénique pharmaceutique pour l'utilisation parentérale selon l'une quelconque des revendications 1, 2, **caractérisée en ce que** les sels peptidiques de l'acide acétique, de l'acide gluconique, de l'acide glucuronique, de l'acide lactique, de l'acide citrique ou de l'acide ascorbique se trouvent dans des solutions en une concentration supérieure à 0,5 mg/ml.

10. Forme galénique pharmaceutique pour l'utilisation parentérale selon l'une quelconque des revendications 1, 2 et 9, **caractérisée en ce qu'**on utilise, comme peptides, le cétrorélix ou l'antarélix dans des solutions en une concentration supérieure à 0,5 mg/ml.

11. Forme galénique pharmaceutique selon l'une quelconque des revendications 1 à 10, **caractérisée en ce que** la libération de la substance active est retardée par l'utilisation de polymères, de préférence d'homopolymères ou de copolymères de l'acide lactique et de l'acide glycolique.

12. Forme galénique pharmaceutique selon l'une quelconque des revendications 1 à 11, **caractérisée en ce qu'**il s'agit, pour les formes galéniques appropriées pour l'utilisation parentérale, de préparations pour injection qui conviennent en particulier pour l'injection sous-cutanée.

13. Forme galénique pharmaceutique pour l'utilisation parentérale selon l'une quelconque des revendications 1, 2 et 9, **caractérisée en ce que** l'antagoniste de la LH-RH est le cétrorélix, **en ce que** le cétrorélix se trouve sous forme de son sel d'acétate, de gluconate ou de glucuronate, **en ce que** cette forme galénique contient en outre de l'acide gluconique ou de l'acide glucuronique sous forme d'acide libre ainsi que le cas échéant d'autres additifs ou adjuvants et se trouve sous forme dissoute ou dispersée dans de l'eau ou dans des mélanges aqueux de solvants.

14. Forme galénique pharmaceutique pour l'utilisation parentérale selon la revendication 13, **caractérisée en ce que** les autres additifs ou adjuvants sont choisis dans le groupe comprenant le mannitol, le 12-(hydroxy)-stéarate de polyéthylèneglycol, le ricinoléate de polyoxyéthylène, la glycine, l'acide citrique et la cyclodextrine, le mannitol étant préféré.

15. Forme galénique pharmaceutique pour l'utilisation parentérale selon la revendication 13 ou 14, **caractérisée en ce que**, pour la préparation de la forme galénique, un lyophilisat, contenant le sel de cétrorélix ainsi que le cas échéant d'autres additifs ou adjuvants, est reconstitué avec un milieu aqueux, contenant de l'acide gluconique ou de l'acide glucuronique sous forme d'acide libre.

16. Procédé pour la préparation d'une forme galénique pharmaceutique selon la revendication 1, **caractérisé en ce que**
(a) on prépare, par changement de sel des sels peptidiques avec de l'acide acétique, de l'acide glucuronique, de l'acide gluconique, de l'acide lactique, de l'acide citrique ou de l'acide ascorbique, les sels correspondants dans un rapport stoechiométrique,
(b) on les dissout dans de l'eau pour injection,
(c) on ajoute le cas échéant des adjuvants selon les revendications 4 à 8,
(d) on filtre ensuite de manière stérile, on transvase dans des flacons d'injection et on lyophilise et
(e) on reconstitue le lyophilisat avant l'administration parentérale.

17. Procédé pour la préparation d'une forme galénique pharmaceutique selon la revendication 11, **caractérisé en ce que**
(au on prépare, par changement de sel des sels peptidiques avec de l'acide acétique, de l'acide glucuronique, de l'acide gluconique, de l'acide lactique, de l'acide citrique ou de l'acide ascorbique, les sels correspondants dans un rapport stoechiométrique,
(b) on incorpore ces sels de manière connue en soi dans des microparticules pour une libération retardée d'homopolymères ou de copolymères de l'acide lactique et de l'acide glycolique et
(c) on met en suspension ces microparticules dans un milieu physiologiquement acceptable destiné à l'injection.

18. Utilisation d'un antagoniste peptidique de la LH-RH pour la préparation d'une forme galénique pharmaceutique appropriée pour l'utilisation parentérale selon l'une quelconque des revendications 1 à 9 ainsi que 11 et 12 pour la prévention et la thérapie de maladies bénignes et malignes dépendant des hormones sexuelles.

19. Utilisation selon la revendication 18, **caractérisée en ce que** l'antagoniste de la LH-RH est choisi dans le groupe constitué par le cétrorélix, l'antarélix, l'antide, l'A-75998, le ganirélix et l'antagoniste Nal-Glu.

20. Utilisation de cétrorélix pour la préparation d'une forme galénique pharmaceutique appropriée pour l'utilisation parentérale selon l'une quelconque des revendications 13 à 15 pour la prévention et la thérapie de maladies bénignes et malignes dépendant des hormones sexuelles.

21. Utilisation selon l'une quelconque des revendications 18 à 20, **caractérisée en ce que** les maladies bénignes et malignes, dépendant des hormones sexuelles sont choisies dans le groupe constitué par l'hyperplasie bénigne de la prostate, le carcinome de la prostate, la puberté précoce, l'hirsutisme, l'hyperplasie de l'endomètre ainsi que ses effets secondaires, le carcinome de l'endomètre, la fécondation in vitro (FIV/SOC/TRA), la contraception, le syndrome prémenstruel (SPM), la myomatose utérine, le cancer du sein, l'obstruction tubaire (PTO), le cancer des ovaires et le carcinome de l'utérus.
